# EUROPEAN PATENT APPLICATION

(11) **EP 4 082 555 A1**
(43) Date of publication of application: **02.11.2022**
(21) Application number: 20907219.8
(22) Date of filing: 24.12.2020
(51) Int. Cl.: A61K 35/744, A61P 3/10, A61P 9/00, A61P 9/14, A23K 10/16, C12N 1/20

(54) **CAPILLARY BLOOD VESSEL DISORDER INHIBITING AGENT, CAPILLARY BLOOD VESSEL DISORDER AMELIORATING AGENT, AND CAPILLARY ANGIOGENESIS ACCELERATING AGENT**

(30) Priority: 27.12.2019 JP 2019239057; 26.05.2020 JP 2020091420
(71) Applicant: National University Corporation Kobe University, Kobe-shi, Hyogo 657-8501 (JP); Kaneka Corporation, Osaka-shi, Osaka 530-8288 (JP)
(72) Inventor: FUJINO Hidemi, Kobe-shi, Hyogo 657-8501 (JP); TATEGAKI Airo, Takasago-shi, Hyogo 676-8688 (JP)
(74) Representative: Hoffmann Eitle
(86) International application number: PCT/JP2020/048408
(87) International publication number: WO 2021/132453

(57) **Abstract**

One or more embodiments of the present invention provide an agent for inhibiting capillary disorder, an agent for improving capillary disorderr, an agent for promoting capillary angiogenesis, or an agent for preventing or inhibiting diabetic complications, which contain microorganisms as active components. One or more embodiments of the present invention relate to an agent for inhibiting capillary disorder an agent for improving capillary disorder, or an agent for promoting capillary angiogenesis, which contain *Enterococcus faecium* R30 strain (Accession No. NITE BP-01362). Another or more embodiments of the present invention relate to an agent for preventing or inhibiting diabetic complications, which contain *Enterococcus faecium* R30 strain.

## Description

### Technical Field

One or more embodiments of the present invention relate to any of an agent for inhibiting capillary disorder, an agent for improving capillary disorder, and an agent for promoting capillary angiogenesis. One or more embodiments of the present invention also relate to an agent for preventing or inhibiting diabetic complications.

### Background Art

It is known that, in diabetic patients, the microvascular network of tissues regresses due to the production of advanced glycation end products and the increase of excess active oxygen associated with an increase in blood glucose level, thereby developing diabetic complications (capillary disorders). Conventionally, it has been considered to be essential for inhibiting and improving diabetic capillary disorders to reduce the blood glucose level.

For example, Patent Literature 1 discloses a hypoglycemic agent and a diabetes treatment and prevention agent containing microorganisms such as *Enterococcus faecalis* as active components. Patent Literature 2 discloses an antihypertensive agent and a hypoglycemic agent containing lactic acid bacterial cells such as *Enterococcus faecalis* as active components.

Meanwhile, the Applicant reports *Enterococcus faecium* R30 strain as lactic acid bacteria having an action of improving fatigue and blood flow, in Patent Literature 3.

### Citation List

### Patent Literature

Patent Literature 1: International Publication No. WO 2005/077390 A
Patent Literature 2: JP Patent Publication (Kokai) No. 10-7577 A
Patent Literature 3: International Publication No. WO 2014/021205 A

### Summary of Invention

### Technical Problem

It can be expected that, if capillary disorders can be inhibited, diabetic complications can be prevented or inhibited. However, Patent Literature 1 and Patent Literature 2 merely disclose that the blood glucose level is reduced or lowered and fail to describe the effect of directly inhibiting or improving capillary disorders. Further, Patent Literature 3 describes only the effect of improving the blood flow as an action of recovering the blood flow that has been decreased under low temperature conditions and then returned in the normal temperature conditions and fails to describe the effect of inhibiting and improving capillary disorders.

It is therefore an object of one or more embodiments of the present invention to provide an agent for inhibiting capillary disorder, an agent for improving capillary disorder, an agent for promoting capillary angiogenesis, and an agent for preventing or inhibiting diabetic complications, which comprise microorganisms as active components.

### Solution to Problem

As a result of diligent studies in order to solve the above problems, the inventor has accomplished the following invention.
(1) An agent for inhibiting capillary disorder comprising *Enterococcus faecium* R30 strain (Accession No. NITE BP-01362).
(2) The agent for inhibiting capillary disorder according to (1), for inhibiting diabetic capillary disorders.
(3) The agent for inhibiting capillary disorder according to (1) or (2), comprising dead bacteria of the strain.
(4) The agent for inhibiting capillary disorder according to any of (1) to (3), wherein the dose of the *Enterococcus faecium* R30 strain (Accession No. NITE BP-01362) is 0.001 mg/kg body weight or more and 1000 mg/kg body weight or less.
(5) The agent for inhibiting capillary disorder according to any of (1) to (4), wherein the content of the *Enterococcus faecium* R30 strain (Accession No. NITE BP-01362) is 0.001 wt% or more and 100 wt% or less.
(6) An agent for improving capillary disorder comprising *Enterococcus faecium* R30 strain (Accession No. NITE BP-01362).
(7) The agent for improving capillary disorder according to (6), for improving diabetic capillary disorders.
(8) The agent for improving capillary disorder according to (6) or (7), comprising dead bacteria of the strain.
(9) The agent for improving capillary disorder according to any of (6) to (8), wherein the dose of the *Enterococcus faecium* R30 strain (Accession No. NITE BP-01362) is 0.001 mg/kg body weight or more and 1000 mg/kg body weight or less.
(10) The agent for improving capillary disorder according to any of (6) to (9), wherein the content of the *Enterococcus faecium* R30 strain (Accession No. NITE BP-01362) is 0.001 wt% or more and 100 wt% or less.
(11) An agent for promoting capillary angiogenesis comprising *Enterococcus faecium* R30 strain (Accession No. NITE BP-01362).
(12) The agent for promoting capillary angiogenesis according to (11), for improving or inhibiting diabetic capillary disorders.
(13) The agent for promoting capillary angiogenesisr according to (11) or (12), comprising dead bacteria of the strain.
(14) The agent for promoting capillary angiogenesis according to any of (11) to (13), wherein the dose of the *Enterococcus faecium* R30 strain (Accession No. NITE BP-01362) is 0.001 mg/kg body weight or more and 1000 mg/kg body weight or less.
(15) The agent for promoting capillary angiogenesis according to any of (11) to (14), wherein the content of the *Enterococcus faecium* R30 strain (Accession No. NITE BP-01362) is 0.001 wt% or more and 100 wt% or less.
(16) An agent for preventing or inhibiting diabetic complications, comprising *Enterococcus faecium* R30 strain (Accession No. NITE BP-01362).
(17) The agent for preventing or inhibiting diabetic complications according to (16), comprising dead bacteria of the strain.
(18) Use of *Enterococcus faecium* R30 strain for the manufacture of an agent for inhibiting capillary disorder.
(19) Use of *Enterococcus faecium* R30 strain for the manufacture of an agent for improving capillary disorder.
(20) Use of *Enterococcus faecium* R30 strain for the manufacture of an agent for promoting capillary angiogenesis.
(21) Use of *Enterococcus faecium* R30 strain for the manufacture of an agent for preventing or inhibiting diabetic complications.
(22) A method for inhibiting capillary disorders, comprising administering an effective amount of *Enterococcus faecium* R30 strain to a patient.
(23) The method according to (22), wherein the patient is a patient in need of inhibiting diabetic capillary disorders.
(24) A method for improving capillary disorders, comprising administering an effective amount of *Enterococcus faecium* R30 strain to a patient.
(25) The method according to (24), wherein the patient is a patient in need of improving diabetic capillary disorders.
(26) A method for promoting capillary angiogenesis, comprising administering an effective amount of *Enterococcus faecium* R30 strain to a patient.
(27) The method according to (26), wherein the patient is a patient in need of promoting capillary angiogenesis.
(28) A method for preventing or inhibiting diabetic complications, comprising administering an effective amount of *Enterococcus faecium* R30 strain to a patient in need of preventing or inhibiting diabetic complications.
(29) Use of *Enterococcus faecium* R30 strain for inhibiting capillary disorders.
(30) The use of (29), comprising administering an effective amount of *Enterococcus faecium* R30 strain to a patient in need of inhibiting capillary disorders.
(31) Use of *Enterococcus faecium* R30 strain for improving capillary disorders.
(32) The use of (31), comprising administering an effective amount of *Enterococcus faecium* R30 strain to a patient in need of improving capillary disorders.
(33) Use of *Enterococcus faecium* R30 strain for promoting capillary angiogenesis.
(34) The use of (33), comprising administering an effective amount of *Enterococcus faecium* R30 strain to a patient in need of promoting capillary angiogenesis.
(35) Use of *Enterococcus faecium* R30 strain for preventing or inhibiting diabetic complications.
(36) The use of (35), comprising administering an effective amount of *Enterococcus faecium* R30 strain to a patient in need of preventing or inhibiting diabetic complications.
(37) The use according to any one of (29) to (36) for non-medical purpose.
(38) The use according to any one of (29) to (36) for medical purpose.
(39) *Enterococcus faecium* R30 strain for use in a method for inhibiting capillary disorders.
(40) The *Enterococcus faecium* R30 strain of (39), wherein the method comprises administering an effective amount of *Enterococcus faecium* R30 strain to a patient in need of inhibiting capillary disorders.
(41) *Enterococcus faecium* R30 strain for use in a method for improving capillary disorders.
(42) The *Enterococcus faecium* R30 strain of (41), wherein the method comprises administering an effective amount of *Enterococcus faecium* R30 strain to a patient in need of improving capillary disorders.
(43) *Enterococcus faecium* R30 strain for use in a method for promoting capillary angiogenesis.
(44) The *Enterococcus ƒaecium* R30 strain of (43), wherein the method comprises administering an effective amount of *Enterococcus ƒaecium* R30 strain to a patient in need of promoting capillary angiogenesis.
(45) *Enterococcus faecium* R30 strain for use in a method for preventing or inhibiting diabetic complications.
(46) The *Enterococcus faecium* R30 strain of (45), wherein the method comprises administering an effective amount of *Enterococcus faecium* R30 strain to a patient in need of preventing or inhibiting diabetic complications.

This description includes the disclosures in Japanese Patent Application No. 2019-239057 and Japanese Patent Application No. 2020-091420, on which the priority of the subject application is based.

### Advantageous Effects of Invention

The agent for inhibiting capillary disorder according to one or more embodiments of the present invention has an excellent effect of inhibiting capillary disorders. The agent for improving capillary disorder or the capillary angiogenesis promoter according to the present invention has an excellent effect of improving capillary disorders or promoting angiogenesis. The agent for inhibiting or improving capillary disorder, or the agent for promoting capillary angiogenesis according to one or more embodiments of the present invention is effective, for example, for inhibiting or improving diabetic capillary disorders in the muscle tissue.

The agent for preventing or inhibiting diabetic complications according to one or more embodiments of the present invention can prevent or inhibit diabetic complications by an action of inhibiting or improving capillary disorders that cause diabetic complications or an action of promoting capillary angiogenesis. The agent for preventing or inhibiting diabetic complications according to one or more embodiments of the present invention can prevent or inhibit diabetic complications by an action different from therapeutic agents for diabetes having hypoglycemic action.

### Brief Description of Drawings

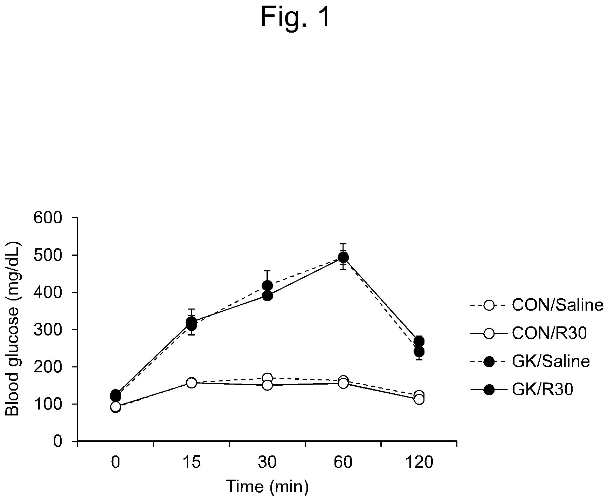

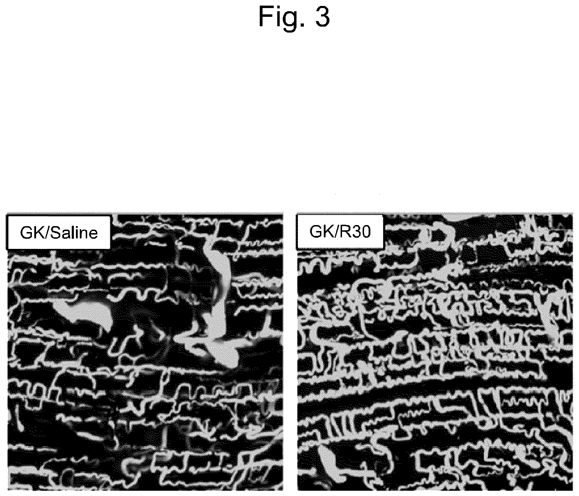

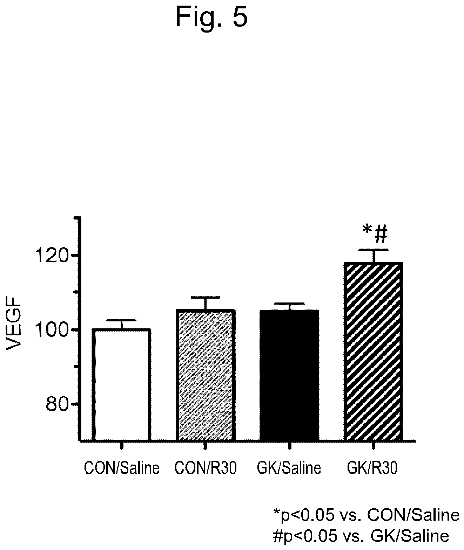

[Figure 1] Figure 1 show the measurement results of blood glucose levels of the Wistar rat/saline-administered group (CON/Saline: n = 6), the Wistar rat/*Enterococcus faecium* R30-administered group (CON/R30: n = 7), the GK rat/saline-administered group (GK/Saline: n = 6), and the GK rat/*Enterococcus faecium* R30-administered group (GK/R30: n = 6) in Example 2, immediately (0 minutes), 15 minutes, 30 minutes, 60 minutes, and 120 minutes after glucose loading.

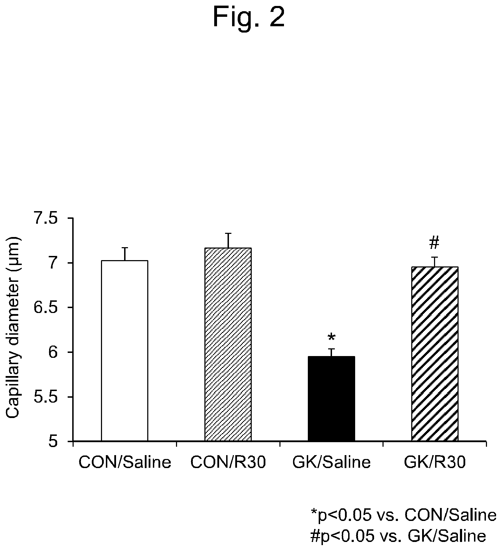

[Figure 2] Figure 2 shows the measurement results of capillary diameters in the soleus muscle of the Wistar rat/saline-administered group (CON/Saline), the Wistar rat/*Enterococcus faecium* R30-administered group (CON/R30), the GK rat/saline-administered group (GK/Saline), and the GK rat/*Enterococcus faecium* R30-administered group (GK/R30) in Example 3 after breeding for 8 weeks.
   ^{∗}p < 0.05 vs. CON/Saline
   #p < 0.05 vs. GK/Saline
[Figure 3] Figure 3 includes three-dimensional images of capillaries of the GK rat/saline-administered group (GK/Saline) and the GK rat/*Enterococcus faecium* R30-administered group (GK/R30) in Example 4 after breeding for 8 weeks, which are constructed by creating longitudinal sections of the soleus muscle using a confocal laser microscope and a fluorescence imaging software.

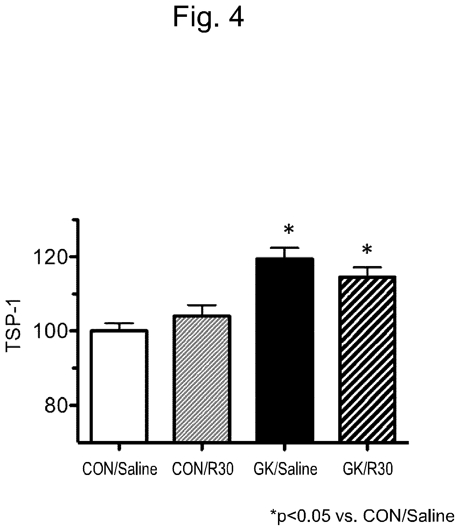

[Figure 4] Figure 4 shows the measurement results of the amounts of angiogenesis inhibitor TSP-1 biosynthesized in the soleus muscle of the Wistar rat/saline-administered group (CON/Saline), the Wistar rat/*Enterococcus faecium* R30-administered group (CON/R30), the GK rat/saline-administered group (GK/Saline), and the GK rat/*Enterococcus faecium* R30-administered group (GK/R30) in Example 5 after breeding for 8 weeks.
   ^{∗}p < 0.05 vs. CON/Saline
[Figure 5] Figure 5 shows the measurement results of the amounts of VEGF that is involved in capillary angiogenesis biosynthesized in the soleus muscle of the Wistar rat/saline-administered group (CON/Saline), the Wistar rat/*Enterococcus faecium* R30-administered group (CON/R30), the GK rat/saline-administered group (GK/Saline), and the GK rat/*Enterococcus faecium* R30-administered group (GK/R30) in Example 5 after breeding for 8 weeks.
   ^{∗}p < 0.05 vs. CON/Saline
   #p < 0.05 vs. GK/Saline

### Description of Embodiments

### <Enterococcus faecium R30 strain>

The agent for inhibiting, improving capillary disorder , the agent for promoting capillary angiogenesis, and the agent for preventing or inhibiting diabetic complications according to one or more embodiments of the present invention comprise *Enterococcus faecium* R30 strain as active components. Hereinafter, *Enterococcus ƒaecium* R30 strain may be abbreviated simply as "R30 strain".

*Enterococcus ƒaecium* R30 strain has been deposited with a depositary institution as follows.
(i) Name and address of depositary institution
   Name: The National Institute of Technology and Evaluation NITE Patent Microorganisms Depositary
   Address: Room 122, Kazusakamatari 2-5-8, Kisarazu, Chiba, Japan
(ii) Domestic contract date: May 16, 2012
(iii) Transfer request receipt date for international deposit: April 24, 2013
(iv) Accession No.: NITE BP-01362

### Enterococcus ƒaecium R30 strain has the following mycological properties.

**[Table 1]**

| Test items | Results | Test items | Results |
|---|---|---|---|
| Gram stainability | Positive | Sugar assimilation | D-ribose + D-mannitol + Lactose + D-Sorbitol - D-trehalose - D-Raffinose - |
| Catalase | Negative | | |
| Spores | None | | |
| Motility | None | | |
| Decomposition of sodium pyruvate | Positive | | |
| Decomposition of esculin | Positive | | |
| Decomposition of pyrrolidonyl-2-naphthylamide | Positive | | |
| Decomposition of 2-naphthyl-β-D-galactopyranoside | Positive | | |
| Decomposition of L-leucine-2-naphthylamide | Positive | | |
| Decomposition of L-alginic acid | Positive | | |

The method for culturing the R30 strain to be used in one or more embodiments of the present invention is not particularly limited as long as the R30 strain can be effectively cultured. For example, it can be cultured by test tube culture, flask culture, and fermentation bath culture. The medium is also not particularly limited, and any medium may be used as long as the R30 strain can be effectively cultured. For example, an MRS medium generally used for culturing lactic acid bacteria can be used.

The R30 strain to be used in one or more embodiments of the present invention may be viable bacteria or dead bacteria (killed bacteria).

Viable bacteria are live R30 strains and include not only those that can grow but also those that are not growing and are dormant but can grow again due to the presence of water or the like.

Dead bacteria (killed bacteria) are bacterial cells that have been sterilized by heating, pressurizing, drug treatment, or the like. The method for sterilization is not particularly limited, and examples thereof can include dry heat sterilization, vapor sterilization, high-pressure vapor sterilization, chemical sterilization, ultrasonic sterilization, electromagnetic wave sterilization, and ultraviolet sterilization. Further, the sterilization may be performed after drying the bacterial cells collected. Further, dead bacteria may be dead bacteria themselves or may be a processed product. Examples of the "processed product" include a processed product obtained by subjecting bacteria to at least one treatment selected from grinding, crushing, concentration, pasting, drying, and dilution. The method for drying bacteria is not particularly limited, and examples thereof can include spray drying, freeze drying, vacuum drying, and drum drying.

### <An agent for inhibiting capillary disorder>

One or more embodiments of the present invention relate to an agent for inhibiting capillary disorder comprising R30 strain.

Another or more embodiments of the present invention relates to use of R30 strain for the manufacture of an agent for inhibiting capillary disorder.

The agent for inhibiting capillary disorder may be a food or drink composition taken for non-medical purposes, a pharmaceutical composition taken for medical purposes, or a feed composition. Specific examples of the food or drink composition, the pharmaceutical composition, or the feed composition are as described below. The pharmaceutical composition may be a pharmaceutical composition for humans or a pharmaceutical composition for nonhuman animals but is preferably a pharmaceutical composition for humans.

Another or more embodiments of the present invention include a method for inhibiting capillary disorders, the method comprising administering an effective amount of *Enterococcus faecium* R30 strain to a patient.

Another or more embodiments of the present invention include use of *Enterococcus faecium* R30 strain for inhibiting capillary disorders.

Another or more embodiments of the present invention include *Enterococcus faecium* R30 strain for use in a method for inhibiting capillary disorders.

The R30 strain to be used in each of the aforementioned embodiments can be in the aforementioned various forms.

The agent for inhibiting capillary disorder according to one or more embodiments of the present invention has an excellent effect of inhibiting capillary disorders. The agent for inhibiting capillary disorder according to one or more embodiments of the present invention is effective, for example, for inhibiting diabetic capillary disorders in the muscle tissue.

The capillary disorders in the present disclosure refer to disorders in which capillaries in tissues regress. Therefore, the term "capillary disorders" is synonymous with "capillary regression" or "tissue capillary regression". The capillary disorders are typically diabetic capillary disorders but may be capillary disorders due to other causes. The diabetic capillary disorders are disorders of the microcapillary network in tissues, particularly caused by the production of advanced glycation end products and the increase in excess reactive oxygen species associated with an increase in blood glucose level, and cause diabetic complications.

Conventionally, it has been considered to be essential for inhibiting diabetic capillary disorders in tissues to control the blood glucose level. In contrast, the agent for inhibiting capillary disorder according to one or more embodiments of the present invention is characterized by inhibiting capillary disorders in tissues without suppressing the increase in blood glucose level. Therefore, in the case where the agent for inhibiting capillary disorder according to one or more embodiments of the present invention and an agent having an action of suppressing the blood glucose level are used in combination and administered to a patient suffering from diabetes and diabetic complications, the dose of the agent having an action of suppressing the blood glucose level can be preferably reduced. The reduction of the dose of the agent having an action of suppressing the blood glucose level can reduce unwanted side effects caused by the agent, leading to a reduction in the cost of the agent, which is useful. Further, if the patient stops the administration of the agent having an action of suppressing the blood glucose level due to side effects, capillary disorders progress over time, and blood is not carried to the extremities, leading to necrosis of the extremities in the worst case. However, the agent for inhibiting capillary disorder of one or more embodiments of the present invention can inhibit capillary disorders without administering the agent having an action of suppressing the blood glucose level. That is, the agent for inhibiting capillary disorder according to one or more embodiments of the present invention can be administered to a patient having side effects against the agent having an action of suppressing the blood glucose level, and is useful since there is no need to limit the patients to be administered.

Further, the administration of the agent for inhibiting capillary disorder according to one or more embodiments of the present invention to a patient can be performed in combination with exercise therapy for the patient.

The patient in the "administration to a patient" may be a patient in need of inhibiting capillary disorders, as will be described later, and examples thereof include those who have not manifested but may develop or manifest the symptoms of capillary disorders in the future, those who need to prevent capillary disorders, and those who want to do so, in addition to patients with capillary disorders.

The action of inhibiting capillary disorders can be evaluated, for example, by measuring the capillary diameter in the soleus muscle of Goto-Kakizaki (GK) rats, which are a spontaneous type 2 diabetes model. If the capillary diameter in the soleus muscle of GK rats to which the test substance is administered is larger than that of laboratory animals to which no test substance is administered, the test substance can be evaluated to have an action of inhibiting capillary disorders. Further, the inhibitory action can be evaluated also by visually observing an image of fluorescently stained capillaries in the soleus muscle of the rats. If an image showing that capillaries of the GK rats to which the test substance is administered are larger than those of laboratory animals to which no test substance is administered can be observed, the test substance can be evaluated to have an action of inhibiting capillary disorders.

The administration frequency and dose of the agent for inhibiting capillary disorder according to one or more embodiments of the present invention to a patient may be appropriately adjusted according to the age, gender, condition, and the like of the patient to be administered. The daily dose of the R30 strain may be appropriately adjusted and can be, for example, 0.001 mg/kg body weight or more and 1000 mg/kg body weight or less. The dose is preferably 0.01 mg/kg body weight or more, more preferably 0.1 mg/kg body weight or more, more preferably 10 mg/kg body weight or more, and is preferably 800 mg/kg body weight or less, more preferably 550 mg/kg body weight or less. The number of administration per day also may be appropriately adjusted and can be, for example, once or more and 5 times or less. The route of administration may be oral administration or parenteral administration but is preferably oral administration. The patient to be administered may be any patient in need of inhibiting capillary disorders and is, for example, a human or a nonhuman animal, preferably, a human. Examples of the nonhuman animal include farm animals, pet animals, and competition animals. The farm animals are not specifically limited, but examples thereof include livestock such as horses, cows, pigs, sheep, goats, camels, and llamas; laboratory animals such as mice, rats, guinea pigs, and rabbits; and poultry such as chickens, ducks, turkeys, and ostriches. The pet animals are not specifically limited, but examples thereof include dogs and cats. The competition animals are not specifically limited, but examples thereof include racehorses. The nonhuman animal is particularly preferably a mammal.

The agent for inhibiting capillary disorder according to one or more embodiments of the present invention can contain various additives such as excipients. Specific examples of the various additives will be described later. Further, the content of the R30 strain in the agent for inhibiting capillary disorder when containing the various additives is not specifically limited, but the lower limit is preferably 0.001 wt% or more, for example, 0.01 wt% or more, more preferably 0.1 wt% or more. The upper limit is preferably 100 wt% or less, for example, 50 wt% or less, more preferably 30 wt% or less.

Further, in the case where the agent for inhibiting capillary disorder according to one or more embodiments of the present invention is in the form of a capsule, a tablet, or the like, the content of the R30 strain in one formulation is not specifically limited and may be appropriately adjusted. The content of the R30 strain in one formulation can be, for example, 0.1 mg/formulation or more and 1000 mg/formulation or less. The content of the R30 strain in one formulation is preferably 5 mg/formulation, 10 mg/formulation, 30 mg/formulation, 50 mg/formulation, or 100 mg/formulation.

In the case where the agent for inhibiting capillary disorder according to one or more embodiments of the present invention is a food or drink composition, a pharmaceutical composition, or a feed composition, or in the case where the agent for inhibiting capillary disorder according to one or more embodiments of the present invention is used for the manufacture of a food or drink composition, a pharmaceutical composition, or a feed composition, the content of the R30 strain in the food or drink composition, the pharmaceutical composition, or the feed composition to be manufactured is not specifically limited and may be appropriately adjusted. The lower limit of the content in the food or drink composition, the pharmaceutical composition, or the feed composition can be, for example, 0.00001 wt% or more. The lower limit is, for example, 0.001 wt% or more, preferably 0.01 wt% or more. The upper limit can be 90 wt% or less. The upper limit is preferably 80 wt% or less, 70 wt% or less, 60 wt% or less, or 50 wt% or less, further preferably 30 wt% or less.

### <An agent for improving capillary disorder>

One or more embodiments of the present invention relate to an agent for improving capillary disorder comprising R30 strain.

Another or more embodiments of the present invention relate to use of R30 strain for the manufacture of an agent for improving capillary disorder.

Like the agent for inhibiting capillary disorder described above, the agent for improving capillary disorder may be a food or drink composition taken for non-medical purposes, a pharmaceutical composition taken for medical purposes, or a feed composition. Specific examples of the food or drink composition, the pharmaceutical composition, or the feed composition are as will be described later. The pharmaceutical composition may be a pharmaceutical composition for human or a pharmaceutical composition for nonhuman animals but is preferably a pharmaceutical composition for human.

Another or more embodiments of the present invention include a method for improving capillary disorders, the method comprising administering an effective amount of *Enterococcus faecium* R30 strain to a patient.

Another or more embodiments of the present invention include use of *Enterococcus faecium* R30 strain for improving capillary disorders.

Another or more embodiments of the present invention include *Enterococcus faecium* R30 strain for use in a method for improving capillary disorders.

The R30 strain to be used in each of the embodiments can be in various forms described above.

The agent for improving capillary disorder according to one or more embodiments of the present invention is effective, for example, for improving diabetic capillary disorders in the muscle tissue.

The agent for capillary disorder according to one or more embodiments of the present invention can improve the capillary disorders accompanied with increase of blood glucose level without taking a diabetes therapeutic agent having an action of suppressing the blood glucose level. Therefore, the agent for improving capillary disorder according to one or more embodiments of the present invention, when used in combination with the diabetes therapeutic agent having an action of suppressing the blood glucose level, is preferable since it can reduce the amount of intake. Further, the agent for improving capillary disorder is useful since it can be administered to a patient who has stopped administration of the diabetes therapeutic agent having an action of suppressing the blood glucose level due to side effects.

Further, the administration of the agent for improving capillary disorder according to one or more embodiments of the present invention to a patient can be performed in combination with exercise therapy for the patient.

The patient in the "administration to a patient" may be a patient in need of improving capillary disorders, and examples thereof include those who have not manifested but may develop or manifest the symptoms of capillary disorders in the future, those who need to prevent capillary disorders, and those who want to do so, in addition to patients with capillary disorders.

The action of improving capillary disorders can be evaluated, for example, by measuring the capillary diameter in the soleus muscle of Goto-Kakizaki (GK) rats, which are a spontaneous type 2 diabetes model. If the capillary diameter of GK rats to which the test substance is administered in the soleus muscle is larger than that of laboratory animals to which no test substance is administered, the test substance can be evaluated to have an action of improving capillary disorders. Further, the improving action can be evaluated also by visually observing an image of fluorescently stained capillaries in the soleus muscle of the rats. If an image showing that capillaries of the GK rats to which the test substance is administered are larger than those of laboratory animals to which no test substance is administered can be observed, the test substance can be evaluated to have an action of improving capillaries.

The administration frequency and dose of the agent for improving capillary disorder according to one or more embodiments of the present invention to the patient can be selected from the examples described above for the agent for inhibiting capillary disorder.

In the case where the agent for improving capillary disorder according to one or more embodiments of the present invention is a food or drink composition, a pharmaceutical composition, or a feed composition, or in the case where the agent for improving capillary disorder according to one or more embodiments of the present invention is used for the manufacture of a food or drink composition, a pharmaceutical composition, or a feed composition, the content of the R30 strain in the food or drink composition, the pharmaceutical composition, or the feed composition can be selected from the examples described above for the agent for inhibiting capillary disorder. In the case where the agent for improving capillary disorder according to one or more embodiments of the present invention is in the form of a capsule, a tablet, or the like, the content of the R30 strain in one formulation can be selected from the examples described above for the agent for inhibiting capillary disorder.

### <An agent for promoting capillary angiogenesis>

One or more embodiments of the present invention relate to an agent for promoting capillary angiogenesis comprising R30 strain.

Another or more embodiments of the present invention relate to use of R30 strain for the manufacture of an agent for promoting capillary angiogenesis.

Like the agent for inhibiting capillary disorder and the agent for improving capillary disorder described above, the agent for promoting capillary angiogenesis may be a food or drink composition taken for non-medical purposes, a pharmaceutical composition taken for medical purposes, or a feed composition. Specific examples of the food or drink composition, the pharmaceutical composition, or the feed composition are as will be described later. The pharmaceutical composition may be a pharmaceutical composition for human or a pharmaceutical composition for nonhuman animals but is preferably a pharmaceutical composition for human.

Another or more embodiments of the present invention include a method for promoting capillary angiogenesis, the method comprising administering an effective amount of *Enterococcus faecium* R30 strain to a patient.

Another or more embodiments of the present invention include use of *Enterococcus faecium* R30 strain for promoting capillary angiogenesis.

Another or more embodiments of the present invention include *Enterococcus faecium* R30 strain for use in a method for promoting capillary angiogenesis.

The R30 strain to be used in each of the embodiments can be in various forms described above.

The agent for promoting capillary angiogenesis according to the present invention is effective, for example, for improving or inhibiting diabetic capillary disorders in the muscle tissue.

The agent for promoting capillary angiogenesis according to one or more embodiments of the present invention can promote angiogenesis and can improve capillary disorders such as capillary regression. Further, the administration of the agent for promoting capillary angiogenesis according to one or more embodiments of the present invention to the patient can be performed in combination with exercise therapy for the patient.

The patient in the "administration to a patient" may be a patient in need of angiogenesis of capillaries, and examples thereof include those who have not manifested but may develop or manifest the symptoms of capillary disorders in the future, those who need to prevent capillary disorders, and those who want to do so, in addition to patients with capillary disorders.

The action of promoting capillary angiogenesis can be evaluated, for example, by measuring the capillary diameter in the soleus muscle of Goto-Kakizaki (GK) rats, which are a spontaneous type 2 diabetes model. If the capillary diameter of GK rats to which the test substance is administered in the soleus muscle is larger than that of laboratory animals to which no test substance is administered, the test substance can be evaluated to have an action of promoting capillary angiogenesis. Further, the promoting action can be evaluated also by visually observing an image of fluorescently stained capillaries in the soleus muscle of the rats. If an image showing that capillaries of the GK rats to which the test substance is administered are larger than those of laboratory animals to which no test substance is administered can be observed, the test substance can be evaluated to have an action of promoting capillary angiogenesis.

The administration frequency and dose of the agent for promoting capillary angiogenesis according to one or more embodiments of the present invention to the patient can be selected from the examples described above for the agent for inhibiting capillary disorder and the agent for improving capillary disorder.

In the case where the agent for promoting capillary angiogenesis according to one or more embodiments of the present invention is a food or drink composition, a pharmaceutical composition, or a feed composition, or in the case where the agent for promoting capillary angiogenesis according to one or more embodiments of the present invention is used for the manufacture of a food or drink composition, a pharmaceutical composition, or a feed composition, the content of the R30 strain in the food or drink composition, the pharmaceutical composition, or the feed composition can be selected from the examples described above for the agent for inhibiting capillary disorder In the case where the agent for promoting capillary angiogenesis according to one or more embodiments of the present invention is in the form of a capsule, a tablet, or the like, the content of the R30 strain in one formulation can be selected from the examples described above for the agent for inhibiting capillary disorder.

<Agent for preventing or inhibiting diabetic complications>

One or more embodiments of the present invention relate to an agent for preventing or inhibiting diabetic complications comprising R30 strain.

Another or more embodiments of the present invention include relates to use of R30 strain for the manufacture of an agent for preventing or inhibiting diabetic complications.

The food or drink composition for preventing or inhibiting diabetic complications may be a food or drink composition taken for non-medical purposes, a food or drink composition taken for medical purposes, or a feed composition. Specific examples of the food or drink composition, the pharmaceutical composition, or the feed composition are as will be described later. The pharmaceutical composition may be a pharmaceutical composition for human or a pharmaceutical composition for nonhuman animals but is preferably a pharmaceutical composition for human.

Another or more embodiments of the present invention include a method for preventing or inhibiting diabetic complications, the method comprising administering an effective amount of R30 strain to a patient in need of preventing or inhibiting diabetic complications.

Another or more embodiments of the present invention include use of *Enterococcus faecium* R30 strain for preventing or inhibiting diabetic complications.

Another or more embodiments of the present invention include *Enterococcus faecium* R30 strain for use in a method for preventing or inhibiting diabetic complications.

The R30 strain to be used in each of the embodiments can be in various forms described above.

In each of the aforementioned embodiments, preventing diabetic complications refers to preventing the development of or affection by diabetic complications in advance, and inhibiting diabetic complications refers to inhibiting the progression of diabetic complications.

The agent for preventing or inhibiting diabetic complications according to one or more embodiments of the present invention can prevent or inhibit diabetic complications by an action of inhibiting capillary disorders that cause diabetic complications. The diabetic complications to be targeted may be type 1 diabetic complications or type 2 diabetic complications.

In the case where the agent for preventing or inhibiting diabetic complications according to one or more embodiments of the present invention and a diabetes therapeutic agent having an action of suppressing the blood glucose level are administered in combination to a patient suffering from diabetes and diabetic complications, the dose of the diabetes therapeutic agent can be preferably reduced. Further, the agent for preventing or inhibiting diabetic complications according to one or more embodiments of the present invention is effective also for a patient who has stopped administration of the diabetes therapeutic agent having an action of suppressing the blood glucose level due to side effects.

The administration of the agent for preventing or inhibiting diabetic complications according to one or more embodiments of the present invention to a patient can be performed in combination with exercise therapy for the patient.

The administration frequency and dose of the agent for preventing or inhibiting diabetic complications according to one or more embodiments of the present invention to the patient in need of preventing or inhibiting diabetic complications may be appropriately adjusted according to the age, gender, condition, and the like of the patient to be administered. The daily dose of the R30 strain may be appropriately adjusted and can be, for example, 0.001 mg/kg body weight or more and 1000 mg/kg body weight or less. The dose is preferably 0.01 mg/kg body weight or more, more preferably 0.1 mg/kg body weight or more, more preferably 10 mg/kg body weight or more, and is preferably 800 mg/kg body weight or less, more preferably 550 mg/kg body weight or less. The number of administration per day also may be appropriately adjusted and can be, for example, once or more and 5 times or less. The route of administration may be oral administration or parenteral administration but is preferably oral administration. The patient to be administered may be any patient in need of inhibiting capillary disorders and is, for example, a human or a nonhuman animal, preferably, a human. Examples of the nonhuman animals are as described above.

In the case where the agent for preventing or inhibiting diabetic complications according to one or more embodiments of the present invention is a food composition, a pharmaceutical composition, or a feed composition, or in the case where the agent for preventing or inhibiting diabetic complications according to one or more embodiments of the present invention is used for the manufacture of a food or drink composition, a pharmaceutical composition, or a feed composition, the content of the R30 strain in the food or drink composition, the pharmaceutical composition, or the feed composition is not specifically limited and may be appropriately adjusted. For example, the content in the food or drink composition, the pharmaceutical composition, or the feed composition can be 0.00001 wt% or more and 50 wt% or less. The ratio is preferably 0.001 wt% or more, more preferably 0.01 wt% or more, further preferably 30 wt% or less.

### <Composition>

The forms of the agent for inhibiting capillary disorder, the agent for improving capillary disorder, the agent for promoting capillary angiogenesis, or the agent for preventing or inhibiting diabetic complications according to one or more embodiments of the present invention are not particularly limited. For example, they may be the R30 strain itself in the various forms described above or a composition comprising R30 strain and other components. The composition comprising R30 strain and other components can be a composition according to the purpose, such as a pharmaceutical composition, a food or drink composition, and a feed composition.

The agent for inhibiting capillary disorder, the agent for improving capillary disorder, the agent for promoting capillary angiogenesis, or the agent for preventing or inhibiting diabetic complications according to one or more embodiments of the present invention exerts a great effect by oral administration, as described below in Examples, and is preferably a pharmaceutical composition, a food or drink composition, or a feed composition to be orally taken or administered, also from the viewpoint of the ease of intake and administration. Hereinafter, "the agent for inhibiting capillary disorder, the agent for improving capillary disorder, the agent for promoting capillary angiogenesis, or the agent for preventing or inhibiting diabetic complications" according to one or more embodiments of the present invention may be simply referred to as an "agent according to one or more embodiments of the present invention".

For example, a composition comprising viable bacteria of the R30 strain can be produced by collecting bacterial cells of the R30 strain in a culture solution cultured by a conventional method, adding a solution in which a protectant has been dissolved to the bacterial cells collected, followed by drying, and then mixing it with a suitable excipient. The protectant refers to a component that protects the bacterial cells from the external environment, maintains the number of viable bacteria, or suppress a decrease in number of viable bacteria. The protectant may be a substance that can reduce the damage to the bacterial cells, such as trehalose, bovine serum albumin, skimmed milk powder, sodium glutamate, L-ascorbic acid, histidine, malic acid, whey, glucose, aspartic acid, methionine, starch, dextrin, sucrose, lactose, sodium chloride, and phosphate. These protectants can be used individually or in combination. The compounding ratio of the protectants is not limited, but the lower limit of the total amount of protectants is preferably 1 wt% or more with respect to the weight of dried bacterial cells. The ratio of 1 wt% or more can reduce the damage of the bacterial cells more reliably. Meanwhile, the upper limit of the ratio is not particularly limited but can be, for example, 50000 wt% or less. The ratio is more preferably 10 wt% or more, furthermore preferably 50 wt% or more, furthermore preferably 100 wt% or more, and more preferably 20000 wt% or less, furthermore preferably 10000 wt% or less.

A composition comprising dead bacteria of the R30 strain can be produced, for example, by collecting bacterial cells in a culture solution cultured by a conventional method, sterilizing the bacterial cells collected, followed by drying, and then mixing it with a suitable excipient.

Further, the agent for inhibiting capillary disorder, the agent for improving capillary disorder, the agent for promoting capillary angiogenesis, or the agent for preventing or inhibiting diabetic complications according to one or more embodiments of the present invention may contain an appropriate amount of various additives that are generally used for the manufacture of a formulation. Examples of the additives include excipients, binders, disintegrants, acidulants, foaming agents, artificial sweeteners, flavors, lubricants, colorants, stabilizers, pH adjusters, and surfactants.

Examples of the excipients include starches such as corn starch, potato starch, flour starch, rice starch, partially gelatinized starch, gelatinized starch, and perforated starch, sugars such as lactose, sucrose, and glucose, sugar alcohols such as mannitol, xylitol, erythritol, sorbitol, and maltitol, and inorganic compounds such as magnesium aluminometasilicate, hydrotalcite, anhydrous calcium phosphate, precipitated calcium carbonate, calcium silicate, and light anhydrous silicic acid.

Examples of the excipients can further include gum arabic, lactose, microcrystalline cellulose, starch, sodium starch glycolate, calcium hydrogen phosphate, magnesium stearate, talc, and colloidal silicon dioxide.

Examples of the binders include hydroxypropyl cellulose, hydroxypropylmethyl cellulose, polyvinylpyrrolidone, gum arabic powder, gelatin, and pullulan.

Examples of the disintegrants include starch, agar, carmellose calcium, sodium carboxymethyl starch, croscarmellose sodium, crospovidone, crystalline cellulose, and F-MELT (trademark, available from Fuji Chemical Industries Co., Ltd.).

Examples of the acidulants include citric acid, tartaric acid, malic acid, and ascorbic acid. Examples of the foaming agents include sodium bicarbonate and sodium carbonate.

Examples of the sweeteners include sodium saccharin, dipotassium glycyrrhizinate, aspartame, stevia, and thaumatin.

Examples of the flavors include lemon oil, orange oil, and menthol.

Examples of the lubricants include magnesium stearate, sucrose fatty acid ester, polyethylene glycol, talc, stearic acid, and sodium stearyl fumarate. Examples of the colorants include edible pigments such as edible yellow No. 5, edible red No. 2, and edible blue No. 2, edible lake pigments, and iron sesquioxide.

Examples of the stabilizers include sodium edetate, tocopherol, and cyclodextrin.

Examples of the pH adjusters include citrate, phosphate, carbonate, tartrate, fumarate, acetate, and amino acid salt.

Examples of the surfactants include polysorbate 80, methylcellulose, hydroxyethyl cellulose, sodium carboxyl methylcellulose, polyoxyethylene sorbitan monolaurate, gum arabic, and powder tragant.

Hereinafter, specific embodiments in which the agent according to one or more embodiments of the present invention is a pharmaceutical composition, a food or drink composition, or a feed composition will be described.

The agent comprising R30 strain according to one or more embodiments of the present invention can be a pharmaceutical composition. The pharmaceutical composition can be in dosage forms such as tablets, powders, capsules, sugar-coated tablets, granules, extract agents, elixirs, syrups, tincture agents, and limonade agents. The pharmaceutical composition can contain additives acceptable as pharmaceutical products (also referred to as pharmaceutically acceptable carriers) depending on the desired dosage form. Examples of the additives can include excipients, disintegrants, lubricants, antioxidants, flavors, seasonings, sweeteners, colorants, thickening stabilizers, color formers, bleaches, fungicides, gum bases, bittering agents, enzymes, brightening agents, acidulants, emulsifiers, enhancers, manufacturing agents, binders, isotonizing agents, buffers, solubilizers, preservatives, stabilizers, aggregation inhibitors, absorption promoters, and coagulants. The pharmaceutical composition can be produced by a conventional method depending on the dosage form.

The pharmaceutical composition may be a pharmaceutical composition to be administered to a human or a pharmaceutical composition to be administered to a nonhuman animal. Specific examples of the nonhuman animal are as described above. The pharmaceutical composition to be administered to a nonhuman animal may be a composition further comprising components acceptable as feeds that can be contained in the later-described feed composition, in addition to the R30 strain as an active component.

The agent comprising R30 strain according to one or more embodiments of the present invention can be used as a food or drink composition. The food or drink composition may be a general food or drink composition such as general foods and beverages or may be a food or drink composition for applications such as so-called health foods, supplements, functional foods, foods with function claims, dietary supplements, foods for specified health uses, foods with nutrient function claims, foods with health function claims, foods for special uses, nursing care foods, smile care foods, chewing/swallowing supplements, concentrated liquid foods, foods for patients, and dietary supplements. The food or drink composition as the agent according to one or more embodiments of the present invention contains components acceptable as food or drink products in addition to the R30 strain.

The forms of the food or drink composition are not specifically limited, and examples thereof include the forms of general food or drink compositions including beverages such as milk beverages, soft drinks, sports drinks, nutrition drinks, beauty drinks, and liquid nutritional supplements; confectioneries such as chewing gums, chocolates, candies, jellies, cakes, pies, cookies, Japanese sweets, snacks, oil confectioneries, rice confectioneries, biscuits, and crackers; frozen confectioneries such as ice creams and frozen desserts; noodles such as Udon, Chinese noodles, spaghetti, and instant noodles; kneaded products such as Kamaboko (fish cakes), Chikuwa (fish cakes), and Hanpen; seasonings such as dressings, mayonnaise, Japanese sauces, and sources; other processed products such as breads, hams, rice porridge, rice, soups, retort foods, frozen foods, edible oil and fat compositions, cooking oils, spraying oils, butter, margarine, shortening, whipped cream, concentrated milk, whitener, dressings, pickle liquids, bakery mix, fried foods, processed meat products, Tofu, and Konnyaku (Konjac); jams, fermented milk, and canned foods. The food or drink composition may be a fermented product obtained by fermenting a general food or drink composition using the R30 strain.

In the case where the food or drink composition is a food for special dietary uses, a food with function claims, or a dietary supplement, the food or drink composition is wrapped in a package, and functions related to inhibition of capillary disorders, improvement of capillary disorders, promotion of capillary angiogenesis, or prevention or inhibition of diabetic complications may be displayed on the package. The package is not particularly limited, but examples thereof include boxes, containers, packaging films, and wrapping paper. Further, examples of the functions to be displayed on the package include not only inhibition of capillary disorders, improvement of capillary disorders, promotion of capillary angiogenesis, prevention or inhibition of diabetic complications, but also functions related thereto, and those disclosed in in this description as the purposes and effects of the action of inhibiting capillary disorders, the action of improving capillary disorders, the action of promoting capillary angiogenesis, and the action of preventing or inhibiting diabetic complications. Of course, the expressions may be different, as long as the functions are similar to these.

Functional foods are foods having an efficacy like that of the agent for inhibiting capillary disorder, which are permitted or designated by the country or public organizations, and examples thereof include health functional foods such as foods with nutrient function claims and foods for specified health uses, and foods for special uses. Although the names and regulations change depending on the situation and the times, those that are essentially the same are included in the present invention.

The food or drink composition as the agent according to one or more embodiments of the present invention may be in a form such as capsules, syrups, tablets, pills, powders, granules, drink agents, injections, infusions, nasal drops, eye drops, suppositories, patches, and sprays. In formulation, a preparation can be formed by appropriately adding other components acceptable as food or drink products such as excipients, disintegrants, lubricants, binders, antioxidants, colorants, aggregation inhibitors, absorption promoters, solubilizers, and stabilizers, in addition to the R30 strain.

The agent according to one or more embodiments of the present invention can be a feed composition (including pet foods). The feed composition contains the R30 strain and components acceptable as feeds to be selected according to the breeding environment such as animal type, growth stage, and area. Examples of the components acceptable as feeds include general feed raw materials, feed additives generally used in compound feeds (such as excipients, extenders, binders, thickeners, emulsifiers, colorants, flavors, food additives, and seasonings), and components other than feed additives (such as antibiotics, bactericides, anthelmintics, and preservatives). Examples of the general feed raw materials include grains or processed grains, chaff and bran, vegetable oil cakes, animal raw materials, minerals, vitamins, amino acids, yeasts such as beer yeast, and fine powder of inorganic substances. Examples of the grains or processed grains include corn, milo, barley, wheat, rye, oats, millet, flour, and wheat germ flour. Examples of the chaff and bran include wheat bran, rice bran, and corn gluten feed. Examples of the vegetable oil cakes include soybean meal, sesame oil cake, cotton seed oil cake, peanut oil cake, sunflower oil cake, and safflower oil cake. Examples of the animal raw materials include skimmed milk powder, fish meal, and meat and bone meal. Examples of the minerals include calcium carbonate, calcium phosphate, salt, and anhydrous silicic acid. Examples of the vitamins include vitamin A, vitamin D, vitamin E, vitamin K, vitamin B1, vitamin B2, vitamin B6, vitamin B12, calcium pantothenate, nicotinamide, and folic acid. Examples of the amino acids include glycine and methionine. Examples of the fine powder of inorganic substances include crystalline cellulose, talc, silica, white mica, and zeolite.

The forms of the feed composition are not specifically limited, and examples thereof include powder, granule, paste, pellet, capsule, and tablet forms. The capsulate may be a hard capsule or a soft capsule. The nonhuman animal to be fed with the feed composition is not specifically limited. Specific examples of the nonhuman animal are as described above.

### [Examples]

### Example 1: Preparation of freeze-dried cells of Enterococcus faecium R30 strain

MRS bouillon (available from KANTO CHEMICAL CO., INC.) (0.52 g) was dissolved in water (10 mL), followed by heat sterilization at 121°C for 15 minutes in an autoclave, to prepare an MRS liquid medium. A glycerol stock suspension (100 µL) of *Enterococcus faecium* R30 strain (Accession No. NITE BP-01362) was inoculated on the liquid medium (10 mL), followed by culture at 37°C for 24 hours, to obtain a preculture solution. The preculture solution (1 mL) was added to the MRS liquid medium (100 mL), followed by further culture at 37°C for 24 hours. After the completion of culture, the culture solution was separated from the bacterial cells by centrifugation at 8000 rpm for 10 minutes. The bacterial cells were washed with sterile water (100 mL), and the sterile water was separated from the bacterial cells by further centrifugation. Sterile water (2 mL) was added to the bacterial cells, followed by heat sterilization at 90°C for 30 minutes and freeze drying, to obtain freeze-dried cells of the *Enterococcus faecium* R30 strain.

### Example 2: Confirmation test for influence of Enterococcus faecium R30 strain on blood glucose level

Thirteen 5-week-old Wistar (CON) rats and twelve 5-week-old male Goto-Kakizaki (GK) rats (type 2 diabetes model animals) were divided into a Wistar rat/saline-administered group (CON/Saline: n = 6), a Wistar rat/*Enterococcus faecium* R30-administered group (CON/R30: n = 7), a GK rat/saline-administered group (GK/Saline: n = 6), and a GK *rat*/*Enterococcus faecium* R30-administered group (GK/R30: n = 6). Each group was preliminary bred for one week with free access to water in addition to a normal feed ("CE-2", available from CLEA Japan, Inc.). Thereafter, the freeze-dried cells of the *Enterococcus faecium* R30 strain produced in Example 1 above were dispersed in saline, which were orally administered to the CON/R30 group and the GK/R30 group using a sonde (oral tube) twice a day, so that the dose of the bacterial cells was 500 mg/kg body weight, and they were bred for 8 weeks.

8 weeks after breeding, diabetes was evaluated by a glucose tolerance test. Diabetes was evaluated by collecting blood 18 hours after fasting (0 minute), orally administering 10 mL of an aqueous glucose solution adjusted to 0.2 g/mL per rat body weight (kg), collecting blood 15, 30, 60, and 120 minutes thereafter, and measuring the blood glucose level in each blood sample with a blood glucose meter ("Precision Xceed", available from Abbott Japan LLC). Figure 1 shows the results of the measurement.

As shown in the results in Figure 1, it turned out from the increase in blood glucose level after glucose administration to the GK/Saline group in which saline was administered to spontaneous type 2 diabetes model GK rats that the GK rats were in a diabetic state. Further, since the GK/R30 group in which the *Enterococcus faecium* R30 strain was administered to GK rats had an increase in blood glucose level after glucose administration, it also turned out that the GK/R30 group was in a diabetic state.

Meanwhile, since the blood glucose level did not increase after glucose administration in the CON/Saline group in which saline was administered to Wistar rats, which were non-diabetes control rats, it turned out that the CON/Saline group was not in a diabetic state, and the CON/R30 group in which the *Enterococcus faecium* R30 strain was administered to Wistar rats was also not in a diabetic state.

### Example 3: Influence of Enterococcus faecium R30 strain on capillary diameters in soleus muscle

In each group of Example 2 above, after the glucose tolerance test performed 8 weeks after breeding, a cannula was inserted into the abdominal aorta under anesthesia with sodium pentobarbital (50 mg/kg), the skeletal muscle capillaries were perfused with saline and then with a fluorescent contrast agent obtained by adding a 1% fluorescent substance (fluorescent marker replenishment ink, available from TOMBOW PENCIL CO., LTD.) to an 8% gelatin solution (available from Sigma-Aldrich Co. LLC.) liquefied at 42°C, and the soleus muscle was then excised. The soleus muscle excised was frozen at -80°C, and a longitudinal section with a thickness of 100 µm was prepared with a cryostat, fixed with 4% paraformaldehyde, and scanned for capillaries present in each 1 µm of muscle section with a confocal laser microscope (C2+, available from NIKON CORPORATION) using an argon laser (488 nm) and a fluorescence imaging software (NIS-Elements, available from NIKON CORPORATION), to measure the capillary diameter. Figure 2 shows the results of the measurement. The number of measurements (n number) of the capillary diameter in the Wistar rat/saline-administered group (CON/Saline) was 29, the n number in the Wistar rat/*Enterococcus faecium* R30-administered group (CON/R30) was 29, the n number in the GK rat/saline-administered group (GK/Saline) was 27, and the n number in the GK rat/*Enterococcus faecium* R30-administered group (GK/R30) was 23.

As shown in the results in Figure 2, it turned out that compared to the capillary diameter of the CON/Saline group (average 7.0 µm), the capillary diameter of the CON/R30 group (average 7.2 µm) was equivalent. Meanwhile, the GK/Saline group in a diabetic state had an average capillary diameter of 6.0 µm, which was significantly reduced as compared with the capillary diameter of the CON/Saline group, and it turned out that capillary disorders had occurred due to the diabetic state. In contrast, the GK/R30 group had an average capillary diameter of 7.0 µm, which was significantly enlarged as compared with the capillary diameter of the GK/Saline group, and it turned out that the capillary diameter was equivalent to that of the CON/Saline group in a non-diabetic state. These results demonstrated that the *Enterococcus faecium* R30 lactic acid bacteria had an effect of inhibiting capillary disorders.

### Example 4: Effect of Enterococcus faecium R30 strain on capillary angiogenesis in soleus muscle

Six 5-week-old male Goto-Kakizaki (GK) rats (type 2 diabetes model animals) were divided into a GK rat/saline-administered group (GK/Saline: n = 3) and a GK rat/*Enterococcus faecium* R30-administered group (GK/R30: n = 3). Each group was preliminary bred for one week with free access to water in addition to a normal feed ("CE-2", available from CLEA Japan, Inc.). Thereafter, the freeze-dried cells of the *Enterococcus faecium* R30 strain produced in Example 1 above were dispersed in saline, which were orally administered to the GK/R30 group using a sonde (oral tube) twice a day, so that the dose of the bacterial cells was 500 mg/kg body weight, and they were bred for 8 weeks. 8 weeks after breeding, a cannula was inserted into the abdominal aorta under anesthesia with sodium pentobarbital (50 mg/kg), the skeletal muscle capillaries were perfused with saline and then with a fluorescent contrast agent obtained by adding a 1% fluorescent substance (fluorescent marker replenishment ink, available from TOMBOW PENCIL CO., LTD.) to an 8% gelatin solution (available from Sigma-Aldrich Co. LLC.) liquefied at 42°C, and the soleus muscle was then excised. The soleus muscle excised was frozen at -80°C, and a longitudinal section with a thickness of 100 µm was prepared with a cryostat, fixed with 4% paraformaldehyde, and scanned in each 1 µm of muscle section with a confocal laser microscope (C2+, available from NIKON CORPORATION) using an argon laser (488 nm) and a fluorescence imaging software (NIS-Elements, available from NIKON CORPORATION), to construct a three-dimensional capillary image. Figure 3 is the three-dimensional image of capillaries constructed with the confocal laser microscope and the fluorescence imaging software.

As shown in Figure 3, the capillary diameter was reduced in the GK/Saline group which is in a diabetic state, and it turned out that capillary disorders had occurred due to the diabetic state. Meanwhile, capillaries were closely arranged in the GK/R30 group as compared with those in the GK/Saline group, and it turned out that new capillaries were formed. This fact revealed that the *Enterococcus faecium* R30 lactic acid bacteria had an effect of improving capillary disorders and/or an effect of promoting capillary angiogenesis.

### Example 5: Confirmation test for capillary angiogenesis

The soleus muscle extracted in Example 3 and frozen at -80°C was homogenized in a HEPES buffer supplemented with a protease inhibitor, and the supernatant was collected by centrifugation. The total amount of protein in the supernatant was measured, the concentration was adjusted to make the total amounts of protein between the samples to be equivalent, and the samples were subjected to SDS-PAGE. The protein developed by electrophoresis was transferred to a PVDF membrane, followed by blocking, a 100-fold diluted solution of anti-thrombospondin-1 (TSP-1) antibody ("sc-59887", available from Santa Cruz Biotechnology), a 200-fold diluted solution of anti-VEGF antibody ("sc-7269", available from Santa Cruz Biotechnology), or a 200-fold diluted solution of anti-β actin antibody ("sc-47778", available from Santa Cruz Biotechnology) was added thereto, followed by incubation, and an HRP-labeled secondary antibody was further added thereto, to develop colors using an HRP detection reagent ("EzWestLumi One", available from ATTO CORPORATION). An image was captured using a lumino-image analyzer ("LAS-1000", available from FUJIFILM Corporation), and the optical density was measured using an image analysis software ("Science Lab", available from FUJIFILM Corporation). Thereby, amounts of TSP-1, VEGF, and β actin biosynthesized were measured, and the amounts of TSP-1 and VEGF biosynthesized in each sample were standardized by the amount of β actin biosynthesized in the same sample and normalized as relative values to the CON/Saline. One-way analysis of variance and Turkey-Kramer multiple comparison test were performed on the measured values. Figure 4 and Figure 5 show the results. In Figure 4 and Figure 5, the symbol "^{∗}" indicates that there was a significant difference of p < 0.05 from the CON/Saline group, and the symbol "#" indicates that there was a significant difference of p < 0.05 from the GK/Saline group. Figure 4 and Figure 5 each show the amounts of TSP-1 and VEGF biosynthesized in each group as relative values when the amounts of TSP-1 and VEGF biosynthesized in the CON/Saline group were each taken as 100.

As shown in Figure 4, it turned out that both the GK/Saline group and the GK/R30 group exhibited a significantly higher expression level of the angiogenesis inhibitor TSP-1 as compared with that of the CON/Saline group, which are non-diabetes control rats. Further, as shown in Figure 5, it turned out that the GK/R30 group exhibited a significantly larger amount of biosynthesized VEGF, which is involved in capillary angiogenesis, as compared to that of the CON/Saline group and the GK/Saline group. This fact revealed that the *Enterococcus faecium* R30 lactic acid bacteria had an effect of promoting capillary angiogenesis in the state where capillary disorders had occurred due to the diabetic state.

All publications, patents and patent applications cited herein are incorporated herein by reference in their entirety.

## Claims

1. An agent for inhibiting capillary disorder comprising *Enterococcus faecium* R30 strain.

2. The agent for inhibiting capillary disorder according to claim 1, for inhibiting diabetic capillary disorders.

3. The agent for inhibiting capillary disorder according to claim 1 or 2, comprising dead bacteria of the strain.

4. An agent for improving capillary disorder comprising *Enterococcus faecium* R30 strain.

5. The agent for improving capillary disorder according to claim 4, comprising dead bacteria of the strain.

6. An agent for promoting capillary angiogenesis comprising *Enterococcus faecium* R30 strain.

7. The agent for promoting capillary angiogenesis according to claim 6, comprising dead bacteria of the strain.

8. An agent for preventing or inhibiting diabetic complications, comprising *Enterococcus faecium* R30 strain.

9. The agent for preventing or inhibiting diabetic complications according to claim 8, comprising dead bacteria of the strain.
